# EUROPEAN PATENT SPECIFICATION

(11) **EP 2 810 674 B1**
(45) Date of publication and mention of the grant of the patent: **15.03.2017**
(21) Application number: 13710441.0
(22) Date of filing: 25.01.2013
(51) Int. Cl.: A61M 5/162, A61M 5/24, A61M 5/28, A61M 5/31, A61J 1/14, A61J 1/20, A61B 5/15

(54) **AN ADAPTER DEVICE FOR REDUCING OR ELIMINATING THE POTENTIAL BACTERIAL CONTAMINATION IN A PROCESS OF EXTRACTION OR TRANSFER OF BLOOD COMPONENTS FROM AT LEAST ONE CONTAINER**
EIN ADAPTER ZUR REDUZIERUNG ODER BESEITIGUNG DES RISIKOS EINER BAKTERIELLEN KONTAMINATION VON BLUTBESTANDTEILEN IN EINEM EXTRAKTIONSPROZESS ODER DURCH TRANSFUSION DIESER AUS ZUMINDEST EINEM BEHÄLTER
DISPOSITIF ADAPTATEUR POUR RÉDUIRE OU ÉLIMINER LE RISQUE DE CONTAMINATION BACTÉRIENNE DANS UN PROCÉDÉ D'EXTRACTION OU DE TRANSFERT DE COMPOSANTS SANGUINS DEPUIS AU MOINS UN CONTENANT

(30) Priority: 30.01.2012 ES 201200084
(43) Date of publication of application: 10.12.2014
(73) Proprietor: Biotechnology Institute, I MAS D.S.L., 01005 - Vitoria (ES)
(72) Inventor: ANITUA ALDECOA, Eduardo, E-01005 - Vitoria (ES)
(74) Representative: Cueto, Sénida
(86) International application number: PCT/ES2013/000017
(87) International publication number: WO 2013/113956

(56) References cited:
- US-A- 4 834 152
- US-A1- 2003 236 497
- US-A1- 2009 216 212

## Description

### Technical field

The invention refers to an adapter which allows and facilitates the simultaneous connection of several containers and reduces or eliminates the potential bacterial contamination in a process of extraction or transfer of blood components from at least one container to a recipient or any applicable destination.

### Prior art

Methods for preparing blood compounds with desirable biological properties, as for example the method disclosed in patent US6569204, carry a certain risk of infection for the patient, as said methods usually involve a first step in which blood is extracted from the patient and/or a latter step of administering the final blood compound to the patient. The risk of contamination arises from the fact that some of the stages of said methods are not immune from bacterial contamination. Bacterial contamination may occur due to a variety of reasons: due to a septicaemia that sends germs into the vascular stream (which may occur when there is need to extract blood from the patient at an initial stage, or when the patient is treated with the final blood compound), due to an inadequate disinfection of the skin at the instant when the vein is punctured (in those methods that require the extraction of blood from the patient), or due to having to manipulate blood and subsequent compounds while the method is being performed.

In regard to the manipulation of blood components, a risk factor of bacterial contamination is the fact that the methods are often performed in open containers, i.e. in containers that are not air-tight or vacuum-sealed. Therefore, in these methods the blood components come into contact with the surrounding air (methods being known as "open circuit" methods). In other cases it may occur that although the method is basically performed in closed containers, it becomes necessary at some point to open a container that contains blood components, thus resulting in a bacterial contamination risk of said blood components. An example of this type of situation is one in which a method is performed in closed containers, but in a given moment it is necessary to open the container in order to transfer part or all of its content to another container. This occurs, for example, when commercial extracting devices are used, usually in the form of syringes, as described in patents ES2364733, US3906947 or US7077826, for extracting blood components from a container. The use of these devices generally requires the opening of the container for introducing the device in order to be able to easily extract the blood components.

In the event that it is necessary to open a container in order to extract part or all of the blood components it contains to a recipient or any other applicable destination, a more secure alternative method of action is known, with regards to the potential bacterial contamination of the blood components. This method is based on having the extraction performed in an appropriate environment (e.g. a laminar flow chamber, a sterile environment or a surgery room). Unfortunately, the installation of special equipment in order to create an appropriate environment often entails a high cost for the user, in terms of adapting the medical installations in order to allow the method to be performed.

US2003236497 describes an adapter device which allows the transfer of a blood test sample to an analyzer without removing said device from the sampler.

The object of the present invention is to provide a cost-effective device which allows and facilitates the simultaneous connection of several containers and reduces the bacterial contamination in a process of extraction or transfer of blood components from the two or more containers to a recipient or any applicable destination (e.g. another container, the body of a syringe or any other extraction device).

Another objective of the invention is to ensure the optimal safety of the user that performs the extraction or transfer from the containers.

### Brief description of the invention

In order to meet at least one of the previous objectives, an adapter device according to claim 1 is proposed for reducing or eliminating the potential bacterial contamination in a process of extraction or transfer of blood components from at least one container, which is provided with a pierceable area, to a recipient or any applicable destination. The device comprises a body that is provided with at least one first area designed to be approached or attached to at least one container, and at least a second area designed to remain accessible to the exterior. At least one needle projects from each first area designed to pierce the pierceable area of a container. The needle presents an inner tube that communicates with an inner tube of the body, which in turn leads into an opening in the second area. The adapter device is also provided with at least one flexible part that maintains each inner tube of the body closed.

The adapter device according to the invention may be connected to two or more containers, in a way that at least one needle pierces the pierceable area of each container. Thus, through the needle's inner tube and the corresponding inner tube of the body of the adapter device, the container's interior is rendered communicable to the exterior, with the exception that a flexible part maintains said communication closed.

The first step to extract or transfer part or all of the content of a container or several containers to a recipient or any applicable destination is to connect the container or containers to the device, in a way that each needle pierces each pierceable area of each container. Since the flexible parts of the device maintain the inner tubes closed, the containers remain sealed (except for an optional venting system as described below) and no potentially contaminating air is able to enter. Next, in order to extract or transfer part or all of the content of each container, the user must simply introduce a transfer or extraction medium into the inner tube of the device and pass it through the inner tube of the needle until it reaches the interior of the container.

Thus, it is not necessary that the extraction device has a sharp tip; instead it will suffice that its tip is able to pass through the flexible part. This guarantees the safety of the user during handling of the device, since there is no risk of being pricked by the tip of the transfer or extraction device.

In addition, as proved, the invention allows the transfer or extraction of components from the containers without the need to open them, and thus guarantees a permanent sealing, which, among other benefits, prevents medical staff from coming in contact with the blood components inside the container, thus reducing the risk of said medical staff being contaminated. An additional advantage is that the step of extracting from the adapter device can be performed by using syringes or other commercial extraction devices, it not being necessary to use a specialized extraction device.

The adapter is manufactured for connecting several containers simultaneously.

### Brief description of the drawings

Details of the invention are depicted in the accompanying figures, which are intended to be illustrative and non-limiting:
- Figure 1 shows a perspective of a first embodiment of the adapter device according to the invention, connected to two containers.
- Figure 2 shows a different perspective of the assembly shown in the previous figure.
- Figure 3 shows a cross-sectional view of part of the assembly shown in the previous figures.
- Figure 4 shows a very schematic front view of a second embodiment of the adapter device according to the invention, about to be connected to three containers.

### Detailed description of the invention

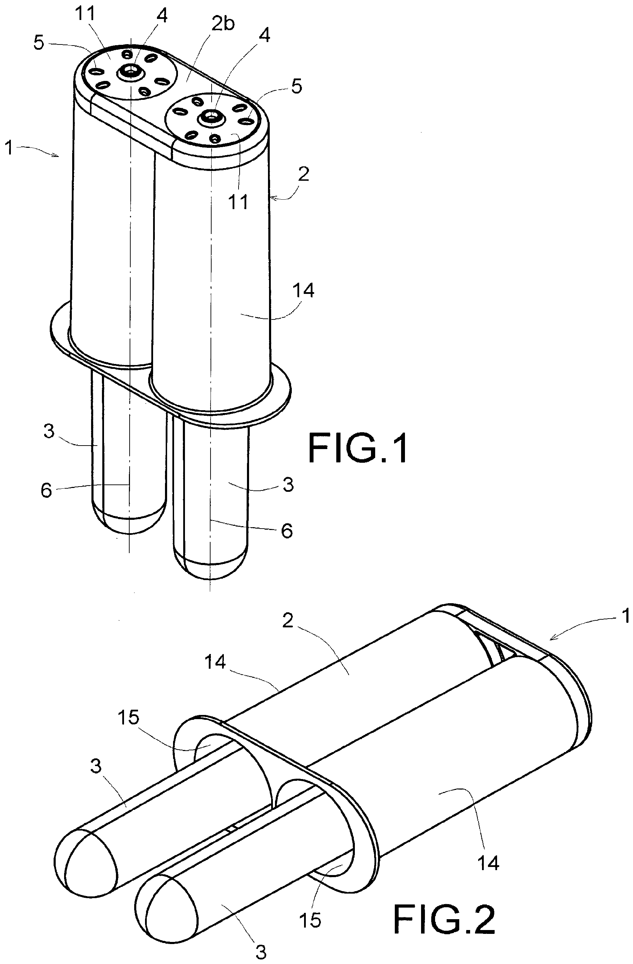

Figures 1 and 2 show two perspectives of a first embodiment of the adapter device according to the invention, which in this case is able to be connected to two containers. As can be observed, the adapter device (1) comprises a body (2) that is attached to two containers (3), which in this embodiment remain partially concealed within the adapter device (1). The exterior area is provided with openings (4) that allow the insertion of extraction or transfer media that will be able to access the interior of the containers (3), as explained below. The exterior area also presents air flow areas (5), the function of which is explained below.

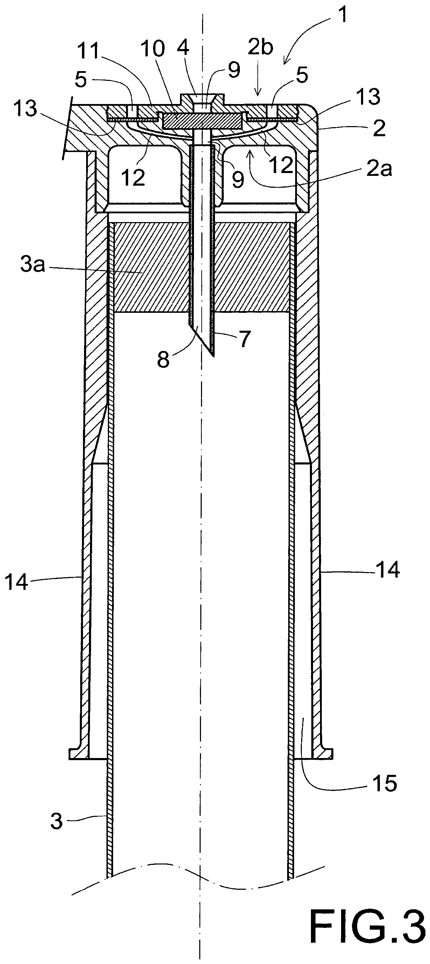

Figure 3 shows a cross-sectional view of part of the assembly shown in the previous figures, according to a section plane containing the longitudinal axes (6) of the containers (3). As can be observed, the body (2) of the adapter device (1) is provided with at least one first area (2a) designed to be approached or attached to at least one container (3) - in this case, two containers - and at least one second area (2b) designed to remain accessible to the exterior. The adapter device (1) also comprises at least one needle (7) that projects from each first area (2a) and that is designed to pierce the pierceable area (3a) of a container (3). The needle (7) presents an inner tube (8) that communicates with an inner tube (9) of the body (2), which in turn leads into an opening (4) in a second area (2b), in a way that the container's interior is rendered communicable to the exterior by means of both tubes (8, 9). In addition, the adapter device (1) comprises at least one flexible part (10) that is arranged so that it closes each inner tube (9) of the body (2). This flexible part (10) prevents the blood components inside the container (3) from being contaminated when said container (3) is attached to the corresponding needle (7) of the adapter device (1).

Thus, when it becomes necessary to extract blood components from the interior of a container (3) that is already attached to the adapter device (1), a needle of an extraction device (e.g. the needle of a syringe) is inserted through the opening (4) and through the flexible part (10); said opening (4) guides the needle of the extraction device towards the inner tube (8) of the needle (7) of the adapter device (1) in a very simple manner. In this process of inserting the extraction device, the flexible part (10) adjusts itself perfectly around the shape of the needle of the extraction device, thus preventing contaminated air from entering into the container (3), ensuring this way that the adapter device (1) remains sealed.

Optionally, the adapter device (1) comprises at least one detachable cover (11), which conceals at least one flexible part (10). By way of example, the embodiment represented in Figures 1 and 2 comprises two covers (11), one for each container (3), that conceal the respective flexible parts (10). The respective flexible parts (10) become visible once the corresponding cover (11) has been removed. This facilitates the fitting of the flexible parts (10) as well as their replacement if needed. Each cover (11) comprises an outlet opening (4) of the inner tube (9) of the body (2) of the adapter device (1).

Optionally, the adapter device (1) comprises a venting system. More specifically, the adapter device (1) comprises at least one air flow area (5, 12) that allows air to pass from the exterior to the inner tube (9) of the body (2). A porous element (13) is located in said air flow area (5, 12) for filtering the air that flows towards the inner tube (9). This venting system allows non-contaminated air to enter the inner tube (9) for maintaining an atmospheric pressure inside said inner tube (9) and the interior of the container (3), thus preventing that the transfer of blood components from the interior of the container (3) is stopped as a result of a decreased pressure in said interior.

In the represented embodiment, the porous element (13) is arranged around the flexible part (10), more specifically as a surrounding ring. This constitutes a simple and effective constructive solution for providing several air flow areas (5) per needle (7).

In this case, in which the porous element (13) is arranged around the flexible part (10), the adapter device (1) may comprise a detachable cover (11) that conceals one or more porous elements (13), which would become visible once the cover (11) is removed. This facilitates the fitting of the porous element (13) as well as its replacement if needed. The cover (11) comprises at least one air flow area (5) arranged towards the porous element (13), allowing the exterior air to enter the interior of the container (3) free of bacterial contamination due to the filtering performed by the porous element (13).

In the represented embodiment, the same cover (11) conceals both the flexible part (10) and the porous element (13) associated with each container (3). This allows optimizing the number of components or parts comprised in the adapter device (1).

In addition, it is preferable that the adapter device (1) comprises at least side walls (14) that define an inner space (15) designed to receive each container (3). The needles (7) remain located inside said inner space (15). The availability of side walls (14) that define said inner spaces (15) for inserting and housing the containers (3) allows for a more intuitive use of the adapter device (1). In addition, the fact that the needles (7) are located inside said inner spaces (15) allows for an increased safety for the user as it lowers the risk of being pricked by the needles (7).

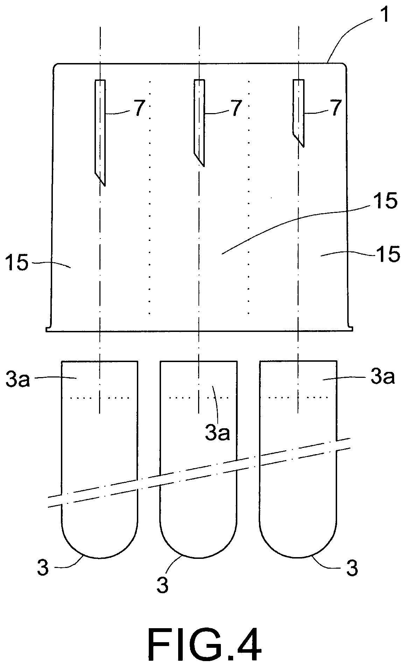

The adapter device (1) is configured to be connected to more than one container, and the needles (7) designed to pierce the pierceable area (3a) of each container (3) present varying lengths for facilitating the connection of the adapter device (1). Figure 4 provides an illustration of this technical option. Said figure shows an adapter device (1) provided with three inner spaces (15) and three respective needles (7), in order to be connected to three containers (3). In the represented embodiment, the needles (7) present a decreasing length so that when the container set (3) is inserted, i.e. when all three containers (3) are inserted at the same time, the pierceable areas (3a) of the containers (3) are not pierced simultaneously but successively. This reduces the force to be exerted by the user on the adapter device (1) for piercing the containers (3).

In addition, the same porous element (13) may be shared by more than one container (3), in a way that the same porous element (13) allows air to pass from the exterior to one or more inner tubes (9) that communicate with their respective needles (7). In this case, an air flow area (12) shall be extended from each respective inner tube (9) to the shared porous element (13).

## Claims

1. An adapter device (1) for reducing or eliminating the potential bacterial contamination in a process of extraction or transfer of blood components from two or more containers (3) to a recipient or any applicable destination, **characterized in that** it comprises:
- a body (2) provided with at least two sets of:
a first area (2a) and a corresponding second area (2b) opposite to said first area (2a), wherein each first area (2a) is capable of being approached or attached to a container (3) and each corresponding second area (2b) is capable of remaining accessible to the exterior, and wherein said second area (2b) comprises an opening (4);
a needle (7) that projects from each first area (2a) and is configured to perforate the perforable area (3a) of a container (3), where the needle (7) presents an inner tube (8) in longitudinal alignment with said opening (4);
an inner tube (9), extending from said opening (4) to said inner tube (8) of said needle (7), providing fluid communication between said opening (4) to said inner tube (8) of said needle (7);
a perforable, sealing flexible part (10) arranged blocking fluid communication between said opening (4) and said inner tube (8) of said needle (7) through said inner tube (9) of the body (2); wherein
- the needles (7) are arranged in a spaced-apart and parallel configuration, and present varying lengths for facilitating the simultaneous connection of the adapter device (1) to at least two containers (3).

2. The adapter device (1) according to claim 1, **characterized in that** each of said at least two sets comprises at least one detachable cover (11) that partially conceals at least one flexible part (10), said at least one flexible part (10) becoming visible once the cover (11) is removed, with said cover (11) comprising said opening (4).

3. The adapter device (1) according to claim 1, **characterized in that** each of said at least two sets further comprises at least one air flow area (5, 12) that allows air to pass from the exterior to the inner tube (9) of the body (2), where in said air flow area (5, 12) is located a porous element (13) for filtering the air that flows towards the inner tube (9).

4. The adapter device (1) according to claim 3, **characterized in that** the porous element (13) is arranged around the flexible part (10).

5. The adapter device (1) according to claim 3, **characterized in that** each of said at least two sets comprises at least one detachable cover (11) that conceals at least one porous element (13), said porous element (13) becoming visible once the cover (11) is removed, with said cover (11) comprising at least one air flow area (5) towards the porous element (13).

6. The adapter device (1) according to claim 5, **characterized in that** the cover (11) further conceals at least one flexible part (10).

7. The adapter device (1) according to claim 1, **characterized in that** each of said at least two sets comprises at least one side wall (14) that defines an inner space (15) designed to receive a container (3), with the needle (7) located inside said inner space (15).

## Patentansprüche

1. Ein Adapter-Gerät (1) zur Reduzierung oder Eliminierung der potenziellen bakteriellen Kontamination bei einem Prozess der Entnahme oder Übertragung von Blutkomponenten von zwei oder mehreren Behältern (3) an einen Empfänger oder ein anwendbares Bestimmungsziel, **dadurch gekennzeichnet, dass** es Folgendes umfasst:
- einen Körper (2), der mit mindestens zwei Sets von Folgendem ausgestattet ist:
einen ersten Bereich (2a) und einen entsprechenden zweiten Bereich (2b) gegenüber besagtem ersten Bereich (2a), wobei jeder erste Bereich (2a) an einen Behälter (3) angenähert oder befestigt werden kann, und jeder entsprechende zweite Bereich (2b) in der Lage ist, nach außen zugänglich zu bleiben, und wobei besagter zweite Bereich (2b) eine Öffnung (4) umfasst;
eine Nadel (7), die aus jedem ersten Bereich (2a) hervorsteht und konfiguriert ist, um den perforierbaren Bereich (3a) eines Behälters (3) zu perforieren, wobei die Nadel (7) ein inneres Rohr (8) in Längsausrichtung mit besagter Öffnung (4) aufweist;
ein inneres Rohr (9), das sich von besagter Öffnung (4) bis zum besagten inneren Rohr (8) der besagten Nadel (7) erstreckt und für Fluidverbindung zwischen besagter Öffnung (4) und besagtem inneren Rohr (8) der besagten Nadel (7) sorgt;
ein perforierbares, abdichtendes flexibles Teil (10), das so angeordnet ist, dass die Fluidverbindung zwischen besagter Öffnung (4) und besagtem inneren Rohr (8) der besagten Nadel (7) durch besagtes inneres Rohr (9) des Körpers (2) blockiert wird; wobei
- die Nadeln (7) in einer räumlich getrennten und parallelen Konfiguration angeordnet sind und unterschiedliche Längen aufweisen, um den gleichzeitigen Anschluss des Adapter-Geräts (1) an mindestens zwei Behältern (3) zu erleichtern.

2. Das Adapter-Gerät (1), gemäß Anspruch 1, **dadurch gekennzeichnet, dass** jedes der besagten mindestens zwei Sets wenigstens einen abnehmbaren Deckel (11) umfasst, der teilweise mindestens ein flexibles Teil (10) abdeckt, wobei besagtes mindestens ein flexibles Teil (10) sichtbar wird, wenn der Deckel (11) entfernt wird, und besagter Deckel (11) besagte Öffnung (4) umfasst.

3. Das Adapter-Gerät (1) gemäß Anspruch 1, **dadurch gekennzeichnet, dass** jedes der besagten mindestens zwei Sets ferner mindestens einen Luftdurchflussbereich (5, 12) umfasst, der ermöglicht, dass Luft von außen in das innere Rohr (9) des Körpers (2) gelangt, wobei sich im besagten Luftdurchflussbereich (5, 12) ein poröses Element (13) zum Filtern der Luft befindet, die zum inneren Rohr (9) hin fließt.

4. Das Adapter-Gerät (1) gemäß Anspruch 3, **dadurch gekennzeichnet, dass** das poröse Element (13) rund um das flexible Teil (10) angeordnet ist.

5. Das Adapter-Gerät (1) gemäß Anspruch 3, **dadurch gekennzeichnet, dass** jedes der besagten mindestens zwei Sets mindestens einen abnehmbaren Deckel (11) umfasst, der mindestens ein poröses Element (13) abdeckt, wobei das besagte poröse Element (13) sichtbar wird, wenn der Deckel (11) entfernt wird, und besagter Deckel (11) mindestens einen Luftdurchflussbereich (5) in Richtung des porösen Elementes (13) umfasst.

6. Das Adapter-Gerät (1) gemäß Anspruch 5, **dadurch gekennzeichnet, dass** der Deckel (11) ferner mindestens ein flexibles Teil (10) abdeckt.

7. Das Adapter-Gerät (1) gemäß Anspruch 1, **dadurch gekennzeichnet, dass** jedes der besagten mindestens zwei Sets mindestens eine Seitenwand (14) umfasst, die einen inneren Raum (15) definiert, der einen Behälter (3) aufnehmen soll, wobei die Nadel (7) sich in besagtem inneren Raum (15) befindet.

## Revendications

1. Un adaptateur (1) pour réduire ou éliminer la contamination bactérienne potentielle dans un procédé d'extraction ou de transfert de composants sanguins de deux conteneurs (3) ou plus vers un récipient ou une destination applicable, **caractérisé par le fait qu'**il comprend :
- un corps (2) muni au moins de deux jeux de :
une première zone (2a) et une seconde zone (2b) correspondante opposée à ladite première zone (2a), où chaque première zone (2a) peut être approchée ou fixée à un conteneur (3) et chaque seconde zone (2b) correspondante peut rester accessible vers l'extérieur, et où ladite seconde zone (2b) comprend une ouverture (4) ;
une aiguille (7) qui se projette à partir de chaque première zone (2a) et est conçue pour perforer la zone perforable (3a) d'un conteneur (3), où l'aiguille (7) présente un tube interne (8) en alignement longitudinal avec ladite ouverture (4) ;
un tube interne (9) s'étendant de ladite ouverture (4) à ledit tube interne (8) de l'aiguille (7), permettant la communication fluide entre ladite ouverture (4) et ledit tube interne (8) de l'aiguille (7);
une partie souple étanche perforable (10) disposée bloquant la communication fluide entre l'ouverture (4) et le tube interne (8) de l'aiguille (7) par le tube interne (9) du corps (2); où
- les aiguilles (7) sont disposées de manière espacée et parallèle, et ont plusieurs longueurs pour faciliter la connexion simultanée de l'adaptateur (1) à au moins deux conteneurs (3).

2. L'adaptateur (1) selon la revendication 1, **caractérisé par le fait que** chacun des deux jeux au moins comprend au moins un couvercle amovible (11) qui cache au moins une pièce flexible (10), cette au moins une pièce flexible (10) étant visible lorsque le couvercle (11) est ouvert, avec ledit couvercle (11) comprenant ladite ouverture (4).

3. L'adaptateur (1) conformément à la revendication 1, **caractérisée par le fait que** chacun des au moins deux jeux comprend également au moins une zone d'écoulement d'air (5, 12) qui permet à l'air de passer de l'extérieur à le tube interne (9) du corps (2), où dans ladite zone d'air (5, 12) est situé un élément poreux (13) pour filtrer l'air qui passe vers le tube interne (9).

4. L'adaptateur (1) conformément à la revendication 3, **caractérisé par le fait que** l'élément poreux (13) est disposé autour de la pièce flexible (10).

5. L'adaptateur (1) selon la revendication 3, **caractérisé par le fait que** chacun des au moins deux jeux comprend au moins un couvercle amovible (11) qui cache au moins un élément poreux (13), ledit élément poreux (13) étant visible lorsque le couvercle (11) est ouvert, avec ledit couvert (11) comprenant au moins une zone de passage d'air (5) vers l'élément poreux (13).

6. L'adaptateur (1) conformément à la revendication 5, **caractérisé par le fait que** le couvercle (11) cache également au moins une partie flexible (10).

7. L'adaptateur (1) conformément à la revendication 1, **caractérisée par le fait que** chacun des au moins deux jeux comprend au moins une paroi latérale (14) qui définit un espace interne (15) conçu pour recevoir un récipient (3), l'aiguille (7) étant située à l'intérieur dudit espace interne (15).
